# EUROPEAN PATENT APPLICATION

(11) **EP 4 666 948 A1**
(43) Date of publication of application: **24.12.2025**
(21) Application number: 24757316.5
(22) Date of filing: 16.02.2024
(51) Int. Cl.: A61B 6/00, A61B 6/03

(54) **METHOD AND MEDICAL IMAGING SYSTEM FOR GENERATING CT COLLATERAL BLOOD FLOW IMAGES THROUGH POST-PROCESSING OF IMAGE INFORMATION OF DYNAMIC CONTRAST-ENHANCED CT AND SIMULTANEOUSLY IMPLEMENTING 3D SUBTRACTION CT ARTERIOGRAPHY, 3D SUBTRACTION CT VENOGRAPHY, AND COLOR-CODED 4D CT ANGIOGRAPHY**

(30) Priority: 16.02.2023 KR 20230020450
(71) Applicant: Deep Clue Inc., Daejeon 34943 (KR)
(72) Inventor: KIM, Hyun Jeong, Daejeon 35248 (KR); ROH, Hong Gee, Seoul 05030 (KR)
(74) Representative: Seemann & Partner Patentanwälte mbB
(86) International application number: PCT/KR2024/095307
(87) International publication number: WO 2024/172599

(57) **Abstract**

The present invention relates to a method, performed by an electronic device, for generating patient-customized CT collateral blood flow images by using blood flow phase segmentation of dynamic contrast-enhanced CT images, and simultaneously implementing 3D subtraction CT arteriography, 3D subtraction CT venography, and color-coded 4D CT angiography.

## Description

The present invention relates to post-processing of dynamic contrast-enhanced CT images, and more particularly, to a method and a medical imaging system for generating CT collateral blood flow images and simultaneously implementing 3D subtraction CT arteriography, 3D subtraction CT venography, and color-coded 4D CT angiography.

### BACKGROUND

Computed tomography (CT), one of the medical imaging devices widely used for diagnosing and evaluating cerebrovascular diseases, is most commonly used along with magnetic resonance imaging (MRI) because of its high penetration rate in hospitals and relatively easy and fast examination.

When evaluating patients with acute ischemic stroke using imaging examinations, the size of early cerebral infarction, detection of occluded blood vessels, evaluation of ischemic penumbra, and evaluation of collateral blood flow and perfusion are important factors.

However, the size of early cerebral infarction cannot be accurately determined with CT images yet, and existing CT collateral blood flow images obtained through repeated CT angiography have low temporal resolution and are obtained only at a certain time after contrast agent injection, so they do not reflect the differences in blood flow depending on the cardiovascular status of each patient. Therefore, evaluation of collateral blood flow through CT collateral blood flow images to date has been limited.

Currently, technologies to generate collateral blood flow images from four-dimensional MR angiography (4D MRA) and perfusion MRI can also be considered; however, for acute stroke patients requiring urgent treatment, CT is preferred over MRI due to its relatively short examination time and easy accessibility.

In conclusion, in cerebrovascular diseases, especially acute cerebral infarction, there is a need to develop CT technology that can evaluate the size of early cerebral infarction and ischemic penumbra, occluded blood vessels, collateral blood flow, and so on more quickly and accurately than existing methods. A technology that can produce more accurate, patient-specific collateral blood flow images and vascular images with distinct separation of arteries and veins through a single dynamic contrast-enhanced CT examination obtained by CT perfusion imaging or a similar method would be one of the optimal solutions for diagnosing cerebrovascular diseases.

### DISCLOSURE

### TECHNICAL PROBLEM

The present invention is directed to providing a method for more easily and conveniently generating CT collateral blood flow images and CT angiography images and an electronic device for performing the same.

The present invention is also directed to providing a method for generating customized CT collateral blood flow images and three-dimensional subtraction CT arteriography, three-dimensional subtraction CT venography, and color-coded four-dimensional CT angiography images according to a patient's cardiovascular condition based on an accurate blood flow phase, and an electronic device for performing the same.

### TECHNICAL SOLUTION

A method, performed by an electronic device, for generating CT collateral blood flow images by using blood flow phase analysis of dynamic contrast-enhanced CT images, and simultaneously implementing 3D subtraction CT arteriography, 3D subtraction CT venography, and color-coded 4D CT angiography according to an exemplary embodiment of the present invention includes loading dynamic contrast-enhanced CT images including dynamic blood flow information; arranging the dynamic contrast-enhanced CT images over time, and generating time-intensity curves (TICs) based on changes in signal intensity of cerebral arteries and cerebral veins of each of the dynamic contrast-enhanced CT images; classifying the dynamic contrast-enhanced CT images according to each blood flow phase by analyzing blood flow phase information determined based on the TICs; generating CT angiography (3D subtraction CT arteriography, 3D subtraction CT venography, and color-coded 4D CT angiography) images from the dynamic contrast-enhanced CT images based on the classified blood flow phases; and segmenting the dynamic contrast-enhanced CT images corresponding to each phase based on the classified blood flow phases, and averaging the CT signal intensities to generate CT collateral blood flow images for each phase.

The loading dynamic contrast-enhanced CT images may include reading contrast-enhanced CT images that contain dynamic blood flow information and anatomical information over time and transforming them into a four-dimensional array.

The loading dynamic contrast-enhanced CT images may include arranging the dynamic contrast-enhanced CT images over time so that they include the dynamic blood flow information and displaying them by transforming them using a maximum intensity projection (MIP) technique.

The generating TICs may include generating a first TIC representing a change in signal intensity of cerebral arteries and a second TIC representing a change in signal intensity of cerebral veins from the dynamic contrast-enhanced CT images.

The classifying according to each blood flow phase may include obtaining blood flow phase information, which divides the blood flow phases into arterial phase, capillary phase, venous phase (early venous phase and late venous phase), and delayed phase according to the magnitude and range of the signal intensity of the cerebral artery and the cerebral vein that change over time.

The generating CT angiography images may include generating CT angiography images including at least one of a 3D subtraction CT arteriography image, a 3D subtraction CT venography image, and a color-coded 4D CT angiography image, from the dynamic contrast-enhanced CT images with their blood flow phases classified.

The generating a 3D subtraction CT arteriography image may include extracting only the arterial signal by subtracting and removing the signal of the venous phase from the signal of the arterial phase.

The generating a 3D subtraction CT venography image may include extracting only the venous signal by subtracting and removing the signal of the arterial phase from the signal of the venous phase.

The generating a color-coded 4D CT angiography image may include generating a color-coded 4D CT angiography image in which blood vessels are sequentially classified according to the order of contrast enhancement by assigning colors according to the blood flow phase.

The color-coded 4D CT angiography may include generating dynamic color CT angiography images in which blood vessels from arteries to capillaries and veins are sequentially classified in the order of contrast enhancement in dynamic contrast-enhanced CT by designating the colors: red for the arterial phase, green for the capillary phase, and blue for the venous phase, and performing color coding that adjusts the color weighting according to the degree of contrast enhancement of blood vessels at each blood flow phase.

An electronic device for generating CT collateral blood flow images by using blood flow phase segmentation of dynamic contrast-enhanced CT images, and simultaneously implementing 3D subtraction CT arteriography, 3D subtraction CT venography, and color-coded 4D CT angiography according to an exemplary embodiment of the present invention includes a processor configured to: load dynamic contrast-enhanced CT images including dynamic blood flow information, arrange the dynamic contrast-enhanced CT images over time, and generate time-intensity curves (TICs) based on changes in signal intensity of cerebral arteries and cerebral veins of each of the dynamic contrast-enhanced CT images, classify the dynamic contrast-enhanced CT images according to each blood flow phase in response to blood flow phase information determined based on the TICs, generate CT angiography images from the dynamic contrast-enhanced CT images based on the classified blood flow phases, and segment the dynamic contrast-enhanced CT images corresponding to each phase based on the classified blood flow phases, and then average the CT signal intensities to generate CT collateral blood flow images for each phase.

The processor may arrange the dynamic contrast-enhanced CT images over time so that they include the dynamic blood flow information and display them by transforming them using a maximum intensity projection (MIP) technique.

The processor may generate a first TIC representing a change in signal intensity of cerebral arteries and a second TIC representing a change in signal intensity of cerebral veins from the dynamic contrast-enhanced CT images.

The processor may obtain blood flow phase information, which divides the blood flow phases into arterial phase, capillary phase, venous phase (early venous phase and late venous phase), and delayed phase according to the magnitude and range of the signal intensities of the cerebral artery and the cerebral vein that change over time.

The processor may generate CT angiography images including at least one of a 3D subtraction CT arteriography image, a 3D subtraction CT venography image, and a color-coded 4D CT angiography image, from the dynamic contrast-enhanced CT images with their blood flow phases classified.

The processor may generate a color-coded 4D CT angiography image in which blood vessels are sequentially classified according to the order of contrast enhancement by assigning colors according to the blood flow phase.

The processor may generate CT collateral blood flow images by averaging the signal intensities of dynamic contrast-enhanced CT images included in each blood flow phase.

The processor may generate the CT collateral blood flow images at each blood flow phase as color images using colors (e.g., red-yellow-green-sky blue-blue, etc.) classified according to the contrast enhancement and signal intensity according to the degree of blood flow.

### ADVANTAGEOUS EFFECT

According to an exemplary embodiment of the present invention, information on one dynamic contrast-enhanced CT scan that can be taken within a short time is obtained by transforming it into images necessary for diagnosing various cerebrovascular diseases through relatively simple interface manipulation. It is possible to simultaneously obtain patient-specific CT collateral blood flow images, 3D subtraction CT arteriography and 3D subtraction CT venography with arteries and veins separated, and color-coded 4D CT angiography using color coding that could not be accurately implemented with existing CT technology, thereby increasing the accuracy and speed of diagnosis of various cerebrovascular diseases including acute stroke.

### BRIEF DESCRIPTION OF THE DRAWINGS

FIG. 1 is a schematic diagram illustrating a process for generating CT vascular images and collateral blood flow images according to an exemplary embodiment of the present invention.
FIG. 2 is a block diagram illustrating the configuration of an electronic device according to an exemplary embodiment of the present invention.
FIG. 3 is a flowchart of the operation of an electronic device according to an exemplary embodiment of the present invention.
FIG. 4 illustrates an interface of a program that implements a method for generating CT vascular images and collateral blood flow images according to an exemplary embodiment of the present invention.
FIG. 5 illustrates transformed images arranged over time according to an exemplary embodiment of the present invention.
FIG. 6 illustrates time-intensity curves according to an exemplary embodiment of the present invention.
FIG. 7 illustrates a process of classifying blood flow phases according to an exemplary embodiment of the present invention.
FIG. 8 illustrates CT angiography images generated according to an exemplary embodiment of the present invention.
FIG. 9 illustrates an interface of a program for controlling the output format and color of CT angiography images generated according to an exemplary embodiment of the present invention.
FIG. 10 illustrates a process of segmenting a CT collateral blood flow image into transverse planes according to an exemplary embodiment of the present invention.
FIGS. 11 and 12 illustrate CT collateral blood flow images generated according to an exemplary embodiment of the present invention.

### BEST MODES FOR CARRYING OUT THE INVENTION

Hereinafter, preferred embodiments according to the present invention will be described in detail with reference to the accompanying drawings. The detailed description to be disclosed hereinafter with the accompanying drawings is intended to describe exemplary embodiments of the present invention and is not intended to represent the only embodiments in which the present invention may be implemented. In the process of clearly explaining the present invention in the drawings, parts unrelated to the present invention may be omitted, and like reference numerals may designate like elements throughout the specification.

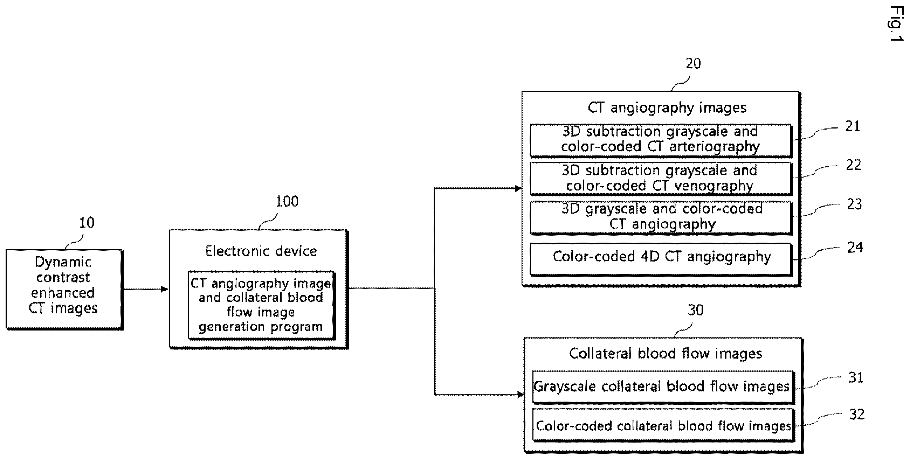

FIG. 1 is a schematic diagram illustrating a process for generating CT angiography images and collateral blood flow images according to an exemplary embodiment of the present invention.

Referring to FIG. 1, an image generation method according to an exemplary embodiment of the present invention inputs and processes dynamic contrast-enhanced CT images 10 into an electronic device 100 to obtain CT angiography images 20 and collateral blood flow images 30.

The electronic device 100 according to an exemplary embodiment of the present invention is a device that generates CT angiography images 20 and collateral blood flow images 30 from dynamic contrast-enhanced CT images 10 by using blood flow phase segmentation of the dynamic contrast-enhanced CT images 10, and may be implemented as a computer, a server, or the like. The electronic device 100 includes a program implementing a method for generating CT angiography images 20 and collateral blood flow images 30.

Dynamic contrast-enhanced CT images 10 according to an exemplary embodiment of the invention means images transformed for program use from a raw CT data image taken while injecting a contrast agent into a blood vessel of a subject (patient, etc.) using CT equipment. In addition, CT angiography images 20 and collateral blood flow images 30 refer to images that are post-processed from dynamic contrast-enhanced CT images 10 to identify the anatomical structure of cerebral blood vessels and the circulatory process of blood flow.

Specifically, CT angiography images 20 include 3D subtraction grayscale and color-coded CT arteriography 21, 3D subtraction grayscale and color-coded CT venography 22, color-coded 3D CT angiography 23, and color-coded 4D CT angiography 24, and the collateral blood flow images 30 include grayscale collateral blood flow images 31 and color-coded collateral blood flow images 32.

As described above, methods for obtaining images to predict collateral blood flow using CT angiography are currently being used, but because images are repeatedly obtained at a predetermined time depending on the CT equipment, it is difficult to accurately evaluate collateral blood flow because the differences in blood flow velocity according to the cardiovascular status of each patient are not reflected. As the accessibility of CT equipment for cerebrovascular disease is relatively superior to that of other imaging equipment and the performance of CT equipment continues to improve, the need for development of cerebrovascular disease evaluation technology utilizing it is increasing.

The present invention proposes a technology that can simultaneously obtain vein-subtraction 3D arteriography, artery-subtraction 3D venography, color-coded 4D angiography, and patient-specific collateral blood flow images with CT by utilizing blood flow phase analysis and segmentation of dynamic contrast-enhanced CT images.

Hereinafter, the configuration and operation of a cerebrovascular disease diagnosis device according to an exemplary embodiment of the present invention will be specifically described with reference to the drawings.

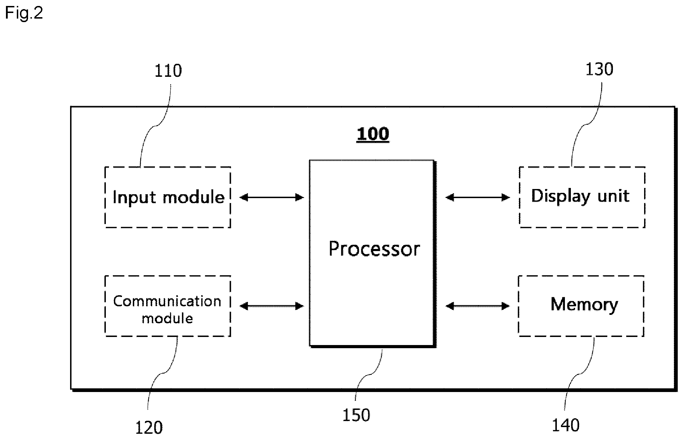

FIG. 2 is a block diagram illustrating the configuration of an electronic device according to an exemplary embodiment of the present invention.

The electronic device 100 according to an exemplary embodiment of the present invention includes an input module 110, a communication module 120, a display 130, a memory 140, and a processor 150.

The input module 110 generates input data in response to a user input of the electronic device 100. For example, the user input may be a user input that initiates the operation of the electronic device 100, a user input that operates an interface of a program that implements a method for generating CT angiography images and collateral blood flow images (hereinafter referred to as the interface), and the user input may be applied without limitation if it is a user input necessary for generating CT angiography images and collateral blood flow images using a patient's CT images.

The input module 110 includes at least one input means. The input module 110 may include a keyboard, a keypad, a dome switch, a touch panel, a touch key, a mouse, a menu button, or the like.

The communication module 120 performs communication with external devices such as a server to receive CT images, blood flow phase information according to signal intensity, etc. To this end, the communication module 120 may perform wireless communication such as 5^{th} generation communication (5G), long term evolution-advanced (LTE-A), long term evolution (LTE), and wireless fidelity (Wi-Fi).

In addition, the communication module 120 can perform wired communication such as a local area network (LAN), a wide area network (WAN), or power line communication, and the communication module 120 can support the establishment of a wired communication channel between the electronic device 100 and an external device, and the performance of communication through the established communication channel.

The display 130 displays display data according to the operation of the electronic device 100. The display 130 may display a screen for loading a transformed image, a screen for displaying a time-intensity curve, a screen for displaying generated CT angiography images and collateral blood flow images, a screen for receiving a user input, etc. In addition to this, any screen that can be displayed while implementing a program, such as an interface may be applied without limitation.

The display 130 includes a liquid crystal display (LCD), a light emitting diode (LED) display, an organic light emitting diode (OLED) display, a micro electro mechanical systems (MEMS) display, and an electronic paper display. The display 130 may be combined with the input module 110 to be implemented as a touch screen.

The memory 140 stores operation programs of the electronic device 100. The memory 140 includes a non-volatile storage for storing data (information) regardless of whether power is supplied or not, and a volatile memory in which data to be processed by the processor 150 is loaded and cannot retain data unless power is provided. The storage includes a flash memory, a hard-disc drive (HDD), a solid-state drive (SSD), a read only memory (ROM), and the like, and the memory includes a buffer and a random access memory (RAM), and the like.

The memory 140 may store CT images, time-intensity curves, blood flow phase information according to the signal intensity of blood vessels, etc. received from an external device, or may store computational programs required in the process of loading CT images, generating time-intensity curves, classifying blood flow phase, generating CT angiography images and collateral blood flow images, etc.

The processor 150 may execute software, such as a program, to control at least one other component (e.g., hardware or software component) of the electronic device 100, and may perform various data processing or computations. According to an embodiment, the processor 150 may include a main processor that acts as a central processing unit (CPU) or application processor and a graphics processing unit (GPU) that can operate independently or together with the main processor.

The processor 150 loads dynamic contrast-enhanced CT images including dynamic blood flow information, arranges the dynamic contrast-enhanced CT images over time, generates time-intensity curves (TICs) based on changes in signal intensity of cerebral arteries and cerebral veins of each of the dynamic contrast-enhanced CT images, analyzes blood flow phase information determined based on the TICs to classify the dynamic contrast-enhanced CT images according to each blood flow phase, generates CT angiography images from the dynamic contrast-enhanced CT images based on the classified blood flow phases, and segments the dynamic contrast-enhanced CT images corresponding to each phase based on the classified blood flow phases, and averages the CT signal intensities to generate CT collateral blood flow images for each phase.

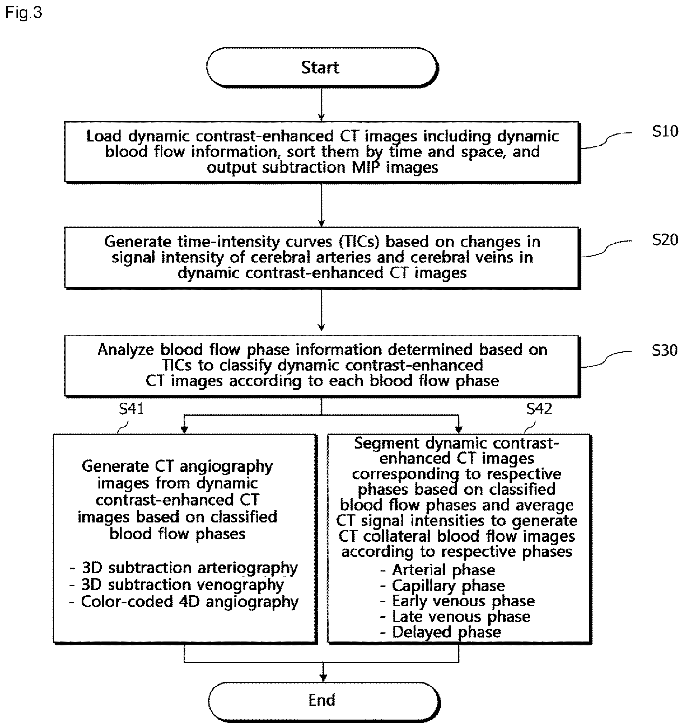

FIG. 3 is a flowchart of the operation of an electronic device according to an exemplary embodiment of the present invention.

According to an exemplary embodiment of the present invention, the processor 150 in FIG. 2 loads dynamic contrast-enhanced CT images including dynamic blood flow information, sort them by time and space, and then outputs subtraction MIP images (S10).

According to an exemplary embodiment of the present invention, the original CT images include high temporal resolution dynamic (multiphase) contrast-enhanced images obtained by a method identical to or similar to CT perfusion imaging.

In this case, the processor 150 sorts the original CT images by time and space so that dynamic blood flow information of blood vessels and tissues can be accurately expressed.

The processor 150 may arrange dynamic contrast-enhanced CT images over time to include dynamic blood flow information and display them by transforming them using the maximum intensity projection (MIP) technique.

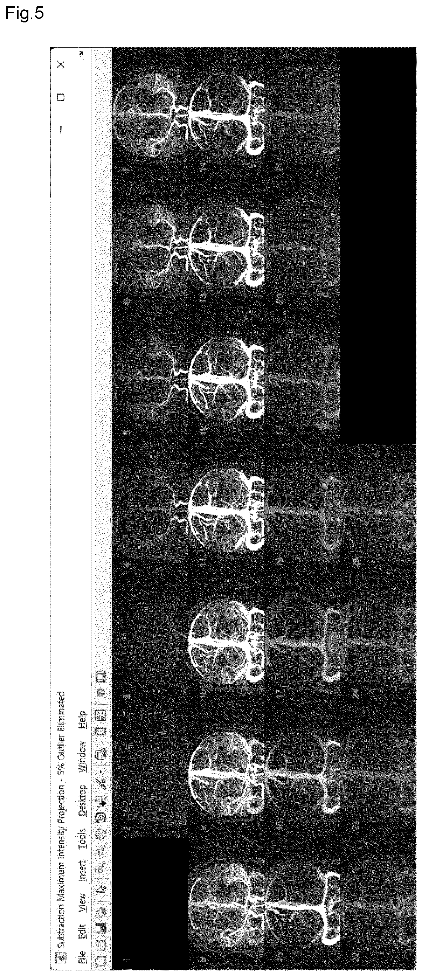

More specifically, the processor 150 may read CT images in DICOM (digital imaging and communications in medicine) format, generate subtraction maximum intensity projection (MIP) vascular images by removing cerebral parenchyma and skull, etc. through variable settings, and arrange the images in chronological order. The subtraction MIP image (hereinafter also referred to as a transformed image) according to an embodiment is illustrated in FIG. 5.

According to an exemplary embodiment of the present invention, the processor 150 generates time-intensity curves (TICs) based on changes in the signal intensity of cerebral arteries and cerebral veins in the dynamic contrast-enhanced CT images (S20).

The processor 150 sorts the dynamic contrast-enhanced CT images in chronological order acquired based on DICOM header information and calculates signal intensities of cerebral arteries and cerebral veins to generate TICs.

The processor 150 may generate a first TIC representing a change in signal intensity of cerebral arteries over time and a second TIC representing a change in signal intensity of cerebral veins over time from dynamic contrast-enhanced CT images.

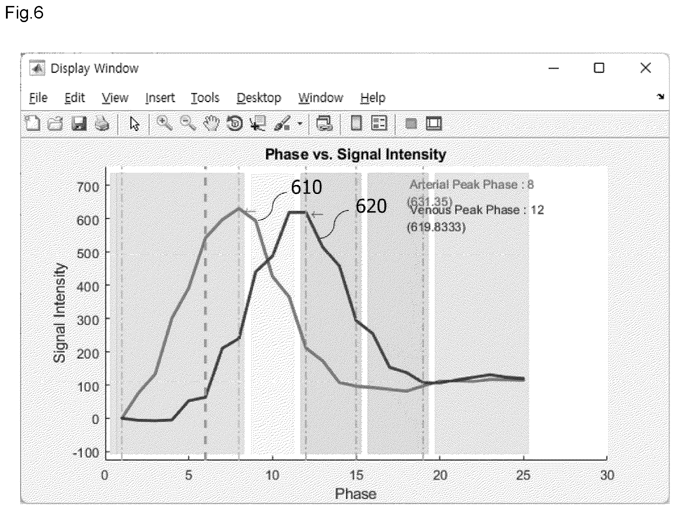

In this case, in order to more accurately classify the blood flow phases, the processor 150 may designate a region of interest (ROI) to each of a cerebral artery (the M1 segment of the middle cerebral artery on the normal side, the A1 segment of the anterior cerebral artery, or the terminal portion of the internal carotid artery) and a cerebral vein (the superior sagittal sinus, the straight sinus, or the distal portion of the large cortical vein), and generate a first TIC and a second TIC based on the signal intensity according to the designated regions of interest. TICs according to an embodiment are illustrated in FIG. 6.

According to an exemplary embodiment of the present invention, the processor 150 analyzes blood flow phase information determined based on TICs to classify dynamic contrast-enhanced CT images according to each blood flow phase (S30).

According to an exemplary embodiment of the present invention, blood flow phase information obtained by analyzing the signal intensity of arteries and veins that changes according to blood flow (hereinafter referred to as "blood flow phase information") refers to information that classifies the blood flow phase into arterial phase, capillary phase, venous phase, and delayed phase based on the magnitude change and range according to the increase and decrease in the signal intensities of the cerebral arteries and the cerebral veins.

For example, the section from the start of the arterial phase, when the signal intensity of the cerebral arteries begins to increase (arterial start phase), to the arterial peak phase when it reaches its highest value is classified as arterial phase, the section between the arterial peak phase and the venous peak phase is classified as capillary phase, the section from the venous peak phase to the point where the signal intensity of the veins decreases and reach plateau (venous end phase) is classified as venous phase, and the phase after the venous phase is classified as delayed phase. In addition, the venous phase may be classified into early venous phase and late venous phase based on its center point.

According to an exemplary embodiment of the present invention, the processor 150 may receive a user input specifying blood flow phase according to the magnitudes and ranges of signal intensities of cerebral arteries and cerebral veins through the input module 110 to obtain blood flow phase information, or may receive predefined blood flow phase information from the outside through the communication module 120.

According to an exemplary embodiment of the present invention, the processor 150 generates CT angiography images from dynamic contrast-enhanced CT images based on the classified blood flow phases (S41).

The processor 150 may generate CT angiography images using the maximum signal intensity of the images included in each blood flow phase. In this case, the blood flow phase may include at least one of the arterial phase, the capillary phase, the early venous phase and late venous phase, and delayed phase, and may include multiple blood flow phases (multi-phases).

The processor 150 may generate CT angiography images including at least one of a 3D subtraction CT arteriography image, a 3D subtraction CT venography image, and a color-coded 4D CT angiography image, from dynamic contrast-enhanced CT images with blood flow phase classified. Specifically, for 3D subtraction CT arteriography, the processor selectively subtracts venous signals from arterial phase signals to generate angiography images showing only arteries, and for 3D subtraction CT venography, the processor selectively subtracts arterial signals from venous phase signals to generate angiography images showing only veins.

The technical details for subtraction are explained in detail during the process of generating each image.

According to an exemplary embodiment of the present invention, the processor 150 segments dynamic contrast-enhanced CT images corresponding to respective phases based on the classified blood flow phases and averages the signal intensities to generate CT collateral blood flow images according to respective phases (S42). There is no fixed order for the S41 and S42 operations, and they may be performed sequentially or in parallel depending on the situation.

Before generating transverse plane collateral blood flow images from dynamic contrast-enhanced CT images, the processor 150 may output a central sagittal plane image and rotate the image so that it is parallel to the anterior commissure-posterior commissure line (AC-PC line) or to a plane preset by the user to obtain an image parallel to other cerebral images such as MRI.

Then, the processor 150 may generate CT collateral blood flow images of slices parallel to a desired plane or a transverse plane, based on the preset image reconstruction information. In this case, the image reconstruction information may include parameters such as slice thickness, inter-slice distance, and the total number of images. The processor 150 may receive a user input for setting image reconstruction information through the input module 110 or generate collateral blood flow images based on preset image reconstruction information.

The processor 150 may generate collateral blood flow images by averaging the signal intensities of images included in their respective blood flow phases. In this case, the blood flow phase is the same as described above in S41.

According to an exemplary embodiment of the present invention, by generating various images simultaneously, the diagnosis and evaluation function of cerebrovascular disease using CT can be improved, thereby increasing the usability of CT equipment and patient convenience.

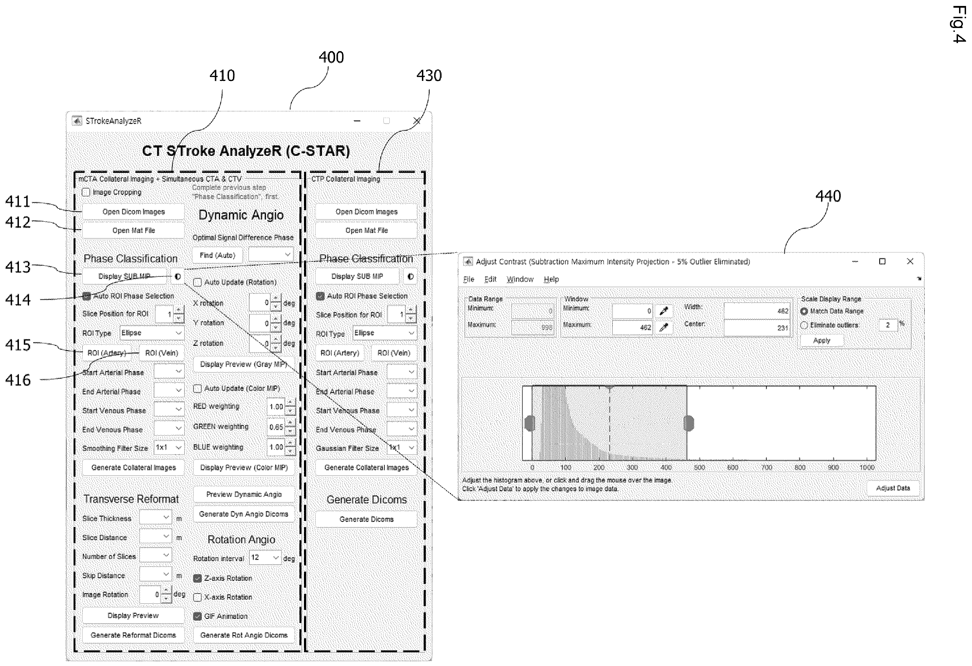

FIG. 4 illustrates an interface of a program that implements a method for generating CT vascular images and collateral blood flow images according to an exemplary embodiment of the present invention.

A series of operations for generating CT angiography images and CT collateral blood flow images described with respect to FIG. 3 may be performed through the interface 400 of FIG. 4. In this case, the design or detailed configuration of the interface 400 is not limited to this drawing and may be adjusted as needed.

The interface 400 according to an exemplary embodiment of the present invention is an interface that uses dynamic contrast-enhanced CT images. This may be divided into a first interface 410 that generates CT angiography images and CT collateral blood flow images using a original CT images of less than 1 mm in slice thickness, and a second interface 430 that generates only CT collateral blood flow images using original CT images reconstructed using slightly thicker slices (e.g., images reconstructed into 1 to 5 mm in slice thickness). However, since the detailed configuration of each interface and the corresponding operation are similar, the description will be based on the first interface 410.

According to an exemplary embodiment of the present invention, a user input for selecting a detailed configuration included in the interface 400 may be received, or the detailed configuration may be selected automatically and sequentially according to a program operation, and the operation method does not limit the present invention.

First, with respect to S10 in FIG. 3, the processor 150 may generate transformed images by inputting (loading) original CT images through icons such as Open Dicom Images 411 and Open Mat File 412.

And through icons such as Display SUB MIP 413, the processor 150 may subtract the cerebral region images of the early phase before the contrast agent reaches the brain from the cerebral region images after the contrast agent reaches the brain, process the images using the MIP technique, sort them in chronological order with appropriate contrast, and output them. Transformed images according to an embodiment are illustrated in FIG. 5.

Furthermore, when the contrast 414 icon is selected, a new window 440 appears, and the processor 150 may go through a process of optimizing the window width and window level after displaying the signal intensity distribution of transformed images as a histogram.

Hereinafter, other configurations and functions will be described with reference to FIGS. 6, 7, and 9.

According to an exemplary embodiment of the present invention, image processing may be performed through simple interface manipulation using dynamic contrast-enhanced CT images.

FIG. 5 illustrates transformed images arranged over time according to an exemplary embodiment of the present invention.

The images shown in FIG. 5 are transformed images containing dynamic blood flow information as described in S10 in FIG. 3, and are arranged in chronological order.

In this case, the processor 150 may assign numbers to each transformed image arranged in chronological order for later classification of blood flow phases.

FIG. 6 illustrates time-intensity curves according to an exemplary embodiment of the present invention.

As described with respect to S20 in FIG. 3, the processor 150 outputs a maximum intensity projection CT images with blood vessels emphasized, sets optimal regions of interest (ROI) for each cerebral artery and cerebral vein in the output image, and then generates time-intensity curves (TICs) of the cerebral artery and cerebral vein based on the changes in signal intensity extracted from each ROI of the cerebral artery and cerebral vein using the ROI (Artery) icon 415 and the ROI (Vein) icon 416 (see interface 400 in FIG. 4).

The processor 150 may generate a first TIC 610 representing a change in signal intensity of cerebral arteries over time in dynamic contrast-enhanced CT images and a second TIC 620 representing a change in signal intensity of cerebral veins over time.

The first TIC 610 and the second TIC 620 in FIG. 6 are generated based on the transformed images from No. 1 (first image) to No. 25 (last image) in FIG. 5. In this case, the x-axis Phase of TIC is generated to correspond to the number of each image, and it is also possible to change it to the time axis. Therefore, it can be seen that the image with the highest signal intensity of the cerebral artery in the first TIC 610 is image number 8 in FIG. 5, and the image with the highest signal intensity of the cerebral vein in the second TIC 620 is image number 12 in FIG. 5.

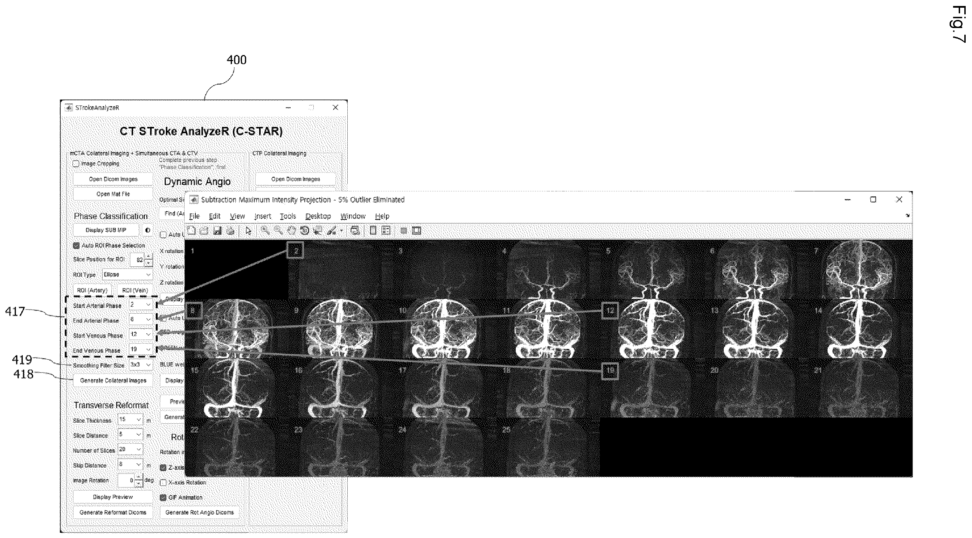

Below, the operation of classifying the blood flow phase using the generated TICs with reference to FIG. 7 is described.

FIG. 7 illustrates a process of classifying blood flow phases according to an exemplary embodiment of the present invention. In this case, the interface 400 of FIG. 7 is identical to the interface 400 of FIG. 4.

As described with respect to S30 in FIG. 3, the processor 150 classifies blood flow phase for composing CT angiography images and CT collateral blood flow images using TICs according to changes in signal intensity of cerebral arteries and cerebral veins.

For example, the processor 150 may identify the image number 417 corresponding to the first TIC 610 and the second TIC 620 of FIG. 6. Referring to FIG. 7, the image numbers corresponding to the start of the arterial phase (Start Arterial phase), the end of the arterial phase (End Arterial phase), the start of the venous phase (Start Venous phase), and the end of the venous phase (End Venous phase) are 2, 8, 12, and 19, respectively, and the processor 150 may generate 418 CT collateral blood flow images based on the classified blood flow phases.

In addition, the processor 150 may select a Gaussian filter size 419 to smooth the CT collateral blood flow images before outputting the images.

Below, CT angiography images are described.

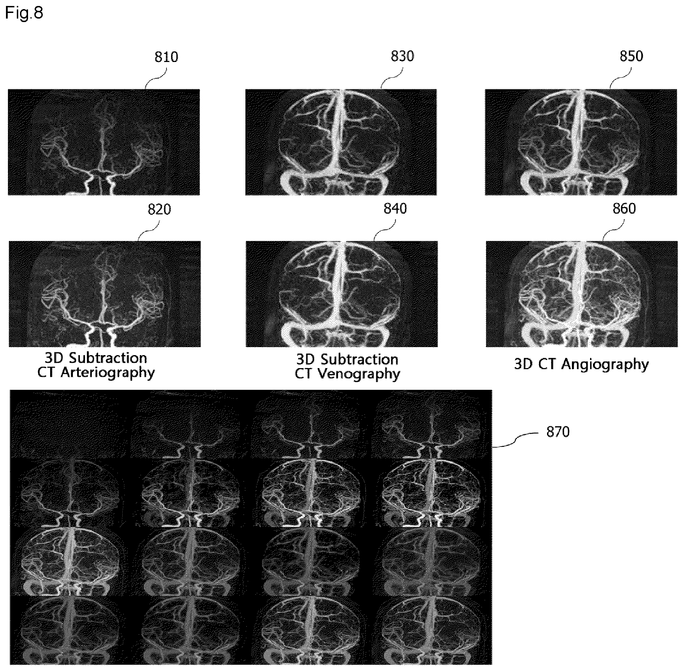

FIG. 8 illustrates CT angiography images generated according to an exemplary embodiment of the present invention.

FIG. 8 illustrates CT angiography images generated through the operation process of FIG. 3. More specifically, FIG. 8 illustrates 3D subtraction CT arteriography 810, 820, 3D subtraction CT venography 830, 840, and 3D CT angiography 850, 860 images in grayscale and color, and color-coded 4D CT angiography images 870 in chronological order.

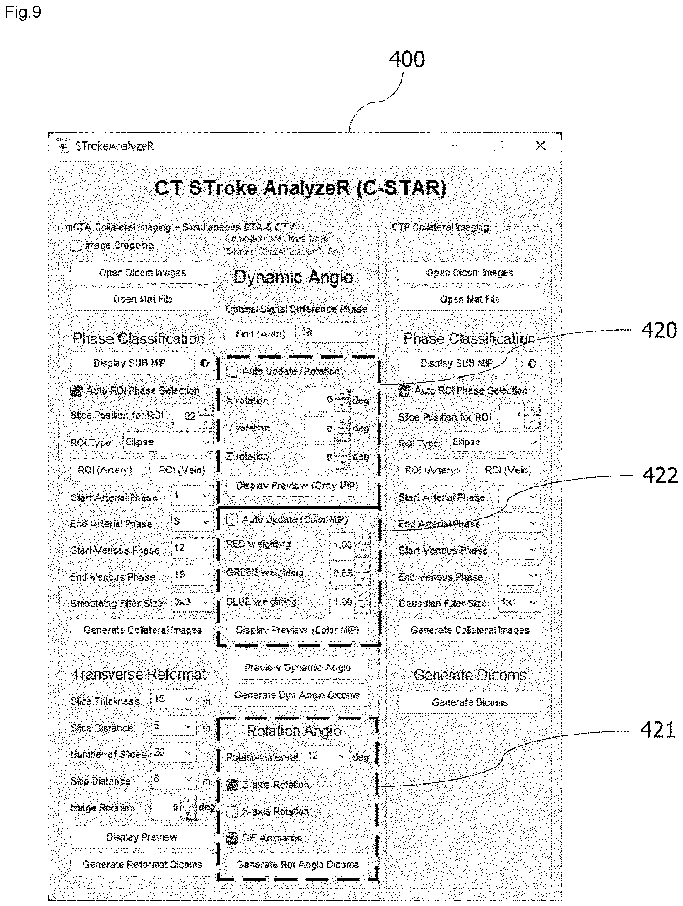

In addition, the interface for generating each CT angiography image is described with reference to FIG. 9.

Hereinafter, a process of generating each image will be described.

The processor 150 classifies and calculates image information of blood vessels from the time when the signal of the artery begins to rise to the arterial peak phase and from the venous peak phase to the time when the signal intensity of the vein decreases and reaches plateau, and image information of blood vessels between the arterial peak phase and the venous peak phase to generate vein-subtraction CT arteriography images 810, 820 and artery-subtraction CT venography images 830, 840.

Specifically, the processor 150 generates an arterial mask having a value greater than or equal to 0 by subtracting a venous phase signal from an arterial phase signal, and updates the arterial phase MIP by a value obtained by multiplying the arterial mask by the arterial phase MIP (maximum intensity projection) to form a 3D subtraction CT arteriography 810, 820. In addition, the processor 150 generates a venous mask by subtracting the arterial phase signal from the venous phase signal and only generating values greater than or equal to 0, and updates the venous phase MIP by a value obtained by multiplying the venous phase MIP by the venous mask to form a 3D subtraction CT venography 830, 840. In this case, in order to optimally subtract the cerebral vein and cerebral artery from 3D subtraction CT arteriography and 3D subtraction CT venography, respectively, an appropriate subtraction reference time point may be determined centering on the time point at which the difference in signal intensity between the cerebral artery and cerebral vein is the greatest (the time point indicated by the gray dotted line in FIG. 6, for example, the 6^{th} phase).

The processor 150 may classify blood vessels in vein-subtraction CT arteriography and artery-subtraction CT venography and 3D and 4D CT angiography with different colors according to the blood flow phase.

For example, the processor 150 displays arterial phase blood vessels from the point in time when the arterial signal begins to rise to the arterial peak phase in red, venous phase blood vessels from the venous peak phase to the point in time when the venous signal intensity decreases and reaches a plateau in blue, and capillary phase blood vessels between the arterial peak phase and the venous peak phase in green. In this case, the color intensities (weighting) of red, blue, and green may be adjusted to better show blood vessels in the desired blood flow phase, so that arteries, veins, and capillaries may be distinguished, and dynamic blood flow changes may be shown in color-coded 4D CT angiography.

FIG. 9 illustrates an interface of a program for controlling the output format and color of CT angiography images generated according to an exemplary embodiment of the present invention.

In this case, the interface 400 of FIG. 9 is identical to the interface 400 of FIGS. 4 and 7.

According to an exemplary embodiment of the present invention, the processor 150 may rotate the image or color the blood vessels to further increase the usability of the image.

To this end, the interface 400 includes portions 420 and 421 for adjusting the rotation of the image and a portion 422 for adjusting the color of the image.

The processor 150 may rotate the CT angiography image by setting angles for the x-axis, y-axis, and z-axis, and may generate a rotational angiography image that shows cerebral blood vessels by continuously rotating by a specific angle (e.g., 12 degrees) based on a particular axis (e.g., the z-axis).

In addition, the processor 150 may specify a color for each blood flow phase to check how the blood flow moves for each phase. In this case, the processor 150 can specify a color by assigning weights to R (red), G (green), and B (blue), and may receive a user input specifying a color through the input module 110.

In addition to this, the color may be specified by selecting one of the preset colors (for example, red for the arterial phase, blue for the venous phase, etc.), and the method of specifying the color does not limit the present invention.

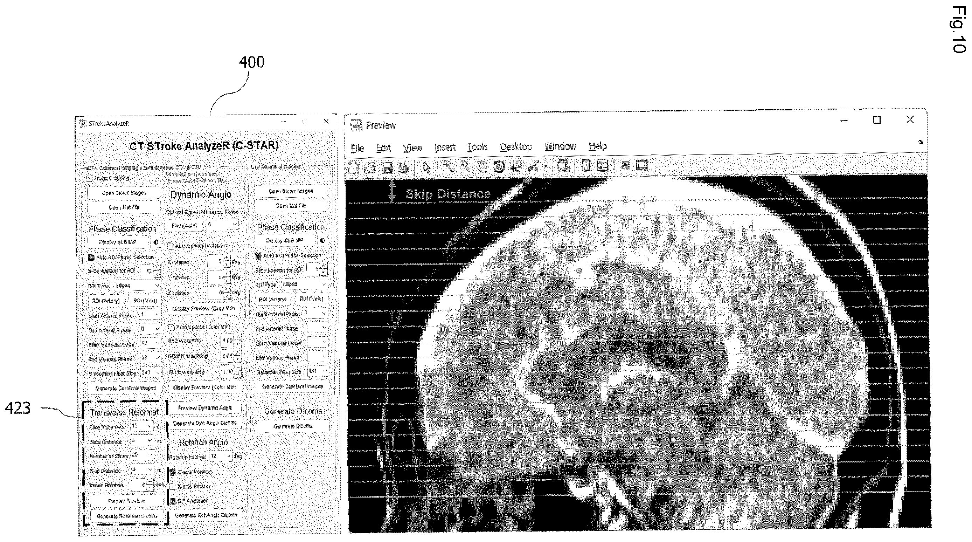

FIG. 10 illustrates a process of reconstructing a CT collateral blood flow image into transverse plane images according to an embodiment of the present invention.

In this case, the interface 400 of FIG. 10 is identical to the interface 400 of FIGS. 4, 7, and 9. The interface 400 may temporarily generate and output a CT image of the central sagittal plane using some of the original CT images, and may first rotate the image so that it is parallel to the anterior commissure-posterior commissure line (AC-PC line) or to a plane set by the user to obtain an image that is parallel to other images.

It includes a portion 423 that transforms to the transverse plane collateral blood flow images based on this.

As described with respect to S42 in FIG. 3, the processor 150 reconstructs a dynamic contrast-enhanced CT images into a transverse plane or a predetermined plane selected by the user based on the preset information, and generates CT collateral blood flow images based on the blood flow phase.

The processor 150 may reconstruct a CT collateral blood flow image into a transverse plane image based on the preset image reconstruction information. In this case, the reconstruction information may include the slice thickness of the images (Slice Thickness), the distance between sections (Slice Distance), and the total number of slices (Number of Slices). However, reconstruction of CT collateral blood flow images is not limited to transverse plane and is possible in multiple cross-sections.

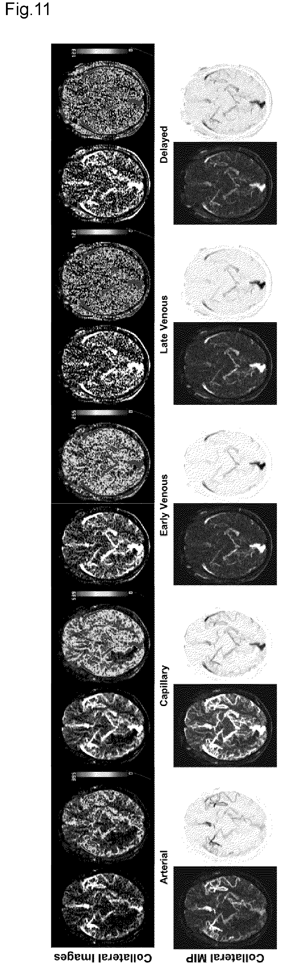

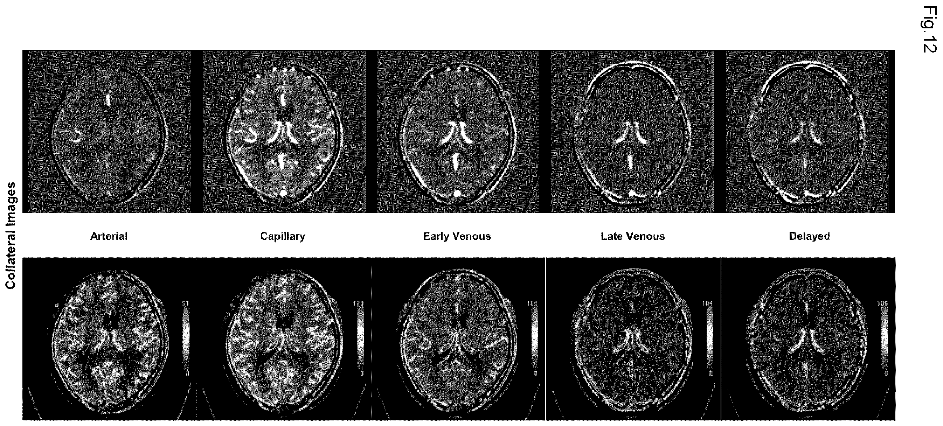

The processor 150 may generate CT collateral blood flow images by averaging the signal intensities of images included in their respective blood flow phases. The generated collateral blood flow images are illustrated in FIGS. 11 and 12.

FIGS. 11 and 12 illustrate collateral blood flow images generated according to an exemplary embodiment of the present invention.

FIG. 11 illustrates CT collateral blood flow images generated using original CT images less than 1 mm in slice thickness, and FIG. 12 illustrates CT collateral blood flow images generated using original CT images reconstructed into slightly thicker slices (5 mm).

In addition, FIGS. 11 and 12 illustrate transverse plane CT collateral blood flow images in the order of blood flow phase: arterial phase, capillary phase, early venous phase, late venous phase, and delayed phase.

According to an exemplary embodiment of the present invention, the processor 150 may generate color-coded images with colors differentiated according to the magnitude of the signal intensity to indicate the degree of blood flow for each phase of the collateral blood flow images.

### [National Research and Development Project that supported this invention]

1.
   [Assignment Unique Number] 1711164322
   [Assignment Number] 2020R1F1A1071619
   [Name of the Ministry] Korea Ministry of Science and ICT
   [Assignment Management (Professional) Organization Name] National Research Foundation of Korea
   [Research Project Name] Individual Basic Research (Korea Ministry of Science and ICT)
   [Research Assignment Name] Development of Imaging Bi-omarkers for prediction of tissue outcome, malignant progression, and hemorrhagic transformation in Acute Ischemic Stroke
   [Contribution rate] 10/100
   [Name of Project Carrying Out Organization] Catholic University of Korea's Industry-Academic Cooperation Foundation
   [Research Period] 2020.06.01-2023.02.28
2.
   [Assignment Unique Number] 1345371257
   [Assignment Number] 00248375 (RS-2023-00248375)
   [Name of the Ministry] Ministry of Education, Republic of Korea
   [Assignment Management (Professional) Organization Name] National Research Foundation of Korea
   [Research Project Name] Establishment of Academic Research Infrastructure in Science and Engineering (Creative and Challenging Research Infrastructure Support Project)
   [Research Assignment Name] Development and Clinical Validation of Artificial Intelligence Technology for Generation and Analysis of CT Collateral Blood Flow Imaging
   [Contribution rate] 20/100
   [Name of Project Carrying Out Organization] Catholic University of Korea's Industry-Academic Cooperation Foundation
   [Research Period] 2023.06.01-2026.05.31
3.
   [Assignment Unique Number] 1711197626
   [Assignment Number] 00252980 (RS-2023-00252980)
   [Name of the Ministry] Korea Ministry of Science and ICT
   [Assignment Management (Professional) Organization Name] National Research Foundation of Korea
   [Research Project Name] Individual Basic Research (Korea Ministry of Science and ICT)
   [Research Assignment Name] Research for Precision Medicine Realization and Patient-Specific Assessment Technology Development for Acute Ischemic Stroke
   [Contribution rate] 20/100
   [Name of Project Carrying Out Organization] Catholic University of Korea's Industry-Academic Cooperation Foundation
   [Research Period] 2023.06.01-2026.02.28
4.
   [Assignment Unique Number] 1465040847
   [Assignment Number] 00266130 (RS-2023-00266130)
   [Name of the Ministry] Ministry of Health and Welfare, Republic of Korea
   [Assignment Management (Professional) Organization Name] Korea Health Industry Development Institute
   [Research Project Name] Clinical Field Problem-Solving Technology Development for Neurological Diseases (R&D)
   [Research Assignment Name] Establishment of Foundation of Development and Commercialization of Software for Collateral Blood Flow Imaging Generation and Analysis
   [Contribution rate] 50/100
   [Name of Project Carrying Out Organization] Catholic University of Korea's Industry-Academic Cooperation Foundation
   [Research Period] 2023.07.01-2026.12.31

## Claims

1. A method, performed by an electronic device, for generating CT collateral blood flow images by using blood flow phase segmentation of dynamic contrast-enhanced CT images, and simultaneously implementing 3D subtraction CT arteriography, 3D subtraction CT venography, and color-coded 4D CT angiography, the method comprising:
loading dynamic contrast-enhanced CT images including dynamic blood flow information;
arranging the dynamic contrast-enhanced CT images over time, and generating time-intensity curves (TICs) based on changes in signal intensities of cerebral arteries and cerebral veins of each of the dynamic contrast-enhanced CT images;
classifying the dynamic contrast-enhanced CT images according to each blood flow phase in response to blood flow phase information determined based on the TICs;
generating CT angiography images from the dynamic contrast-enhanced CT images based on the classified blood flow phases; and
segmenting the dynamic contrast-enhanced CT images corresponding to each phase based on the classified blood flow phases, and averaging the CT signal intensities to generate CT collateral blood flow images for each phase.

2. The method of claim 1,
wherein the loading dynamic contrast-enhanced CT images comprises:
arranging the dynamic contrast-enhanced CT images over time so that they include the dynamic blood flow information and displaying them by transforming them using a maximum intensity projection (MIP) technique to emphasize blood vessels.

3. The method of claim 1,
wherein the generating TICs comprises:
generating a first TIC representing a change in signal intensity of cerebral arteries over time and a second TIC representing a change in signal intensity of cerebral veins over time from the dynamic contrast-enhanced CT images.

4. The method of claim 3,
wherein the classifying the blood flow phases based on the TICs comprises:
obtaining blood flow phase information, which divides the blood flow phases into arterial phase, capillary phase, early venous phase, late venous phase, and delayed phase according to the magnitude and range of the signal intensities of the cerebral artery and the cerebral vein that change over time.

5. The method of claim 4,
wherein the generating CT angiography images comprises:
generating CT angiography images including at least one of a 3D subtraction CT arteriography image, a 3D subtraction CT venography image, and a color-coded 4D CT angiography image, from the dynamic contrast-enhanced CT images with their blood flow phases classified.

6. The method of claim 5,
wherein the generating a 3D subtraction CT arteriography image comprises:
extracting only the arterial signal by subtracting and removing the signal of the venous phase from the signal of the arterial phase.

7. The method of claim 5,
wherein the generating a 3D subtraction CT venography image comprises:
extracting only the venous signal by subtracting and removing the signal of the arterial phase from the signal of the venous phase.

8. The method of claim 5,
wherein the generating a color-coded 4D CT angiography image comprises:
generating a color-coded 4D CT angiography image in which blood vessels are sequentially classified according to the order of contrast enhancement by assigning colors according to the blood flow phase.

9. The method of claim 1,
wherein the generating CT collateral blood flow images comprises:
generating CT collateral blood flow images by averaging the signal intensities of dynamic contrast-enhanced CT images included in each blood flow phase.

10. An electronic device for generating CT collateral blood flow images by using blood flow phase segmentation of dynamic contrast-enhanced CT images, and simultaneously implementing 3D subtraction CT arteriography, 3D subtraction CT venography, and color-coded 4D CT angiography, the electronic device comprising:
a processor configured to:
load dynamic contrast-enhanced CT images including dynamic blood flow information,
arrange the dynamic contrast-enhanced CT images in time order, and generate time-intensity curves (TICs) based on changes in signal intensity of cerebral arteries and cerebral veins of each of the dynamic contrast-enhanced CT images,
classify the dynamic contrast-enhanced CT images according to each blood flow phase in response to blood flow phase information determined based on the TICs,
generate CT angiography images from the dynamic contrast-enhanced CT images by analyzing the classified blood flow phases, and
segment the dynamic contrast-enhanced CT images corresponding to each phase based on the classified blood flow phases, and average the CT signal intensities to generate CT collateral blood flow images for each phase.

11. The electronic device of claim 10,
wherein the processor is configured to:
arrange the dynamic contrast-enhanced CT images in time order so that they include the dynamic blood flow information and display them by transforming them using a maximum intensity projection (MIP) technique to emphasize blood vessels.

12. The electronic device of claim 10,
wherein the processor is configured to:
generate a first TIC representing a change in signal intensity of cerebral arteries over time and a second TIC representing a change in signal intensity of cerebral veins over time from the dynamic contrast-enhanced CT images.

13. The electronic device of claim 12,
wherein the processor is configured to:
obtain blood flow phase information, with which the blood flow phases are divided into arterial phase, capillary phase, early venous phase, late venous phase, and delayed phase according to the magnitude and range of the signal intensities of the cerebral artery and the cerebral vein that change over time.

14. The electronic device of claim 13,
wherein the processor is configured to:
generate CT angiography images including at least one of a 3D subtraction CT arteriography image, a 3D subtraction CT venography image, and a color-coded 4D CT angiography image, from the dynamic contrast-enhanced CT images with their blood flow phases classified based on the TICs.

15. The electronic device of claim 14,
wherein the processor is configured to:
generate a color-coded 4D CT angiography image in which blood vessels are sequentially classified according to the order of contrast enhancement by assigning colors according to the blood flow phase.

16. The electronic device of claim 10,
wherein the processor is configured to:
generate CT collateral blood flow images by averaging the signal intensities of dynamic contrast-enhanced CT images included in each blood flow phase.
